# EUROPEAN PATENT APPLICATION

(11) **EP 0 700 994 A1**
(43) Date of publication of application: **13.03.1996**
(21) Application number: 94930363.0
(22) Date of filing: 25.10.1994
(51) Int. Cl.: C12N 15/06, C12P 21/08, C12N 5/20

(54) **ANTI-IGF-I MONOCLONAL ANTIBODY**

(30) Priority: 23.03.1994 JP 89073/94
(71) Applicant: JAPAN CLINICAL LABORATORIES, Kuze-gun, Kyoto 613 (JP)
(72) Inventor: TERANO, Yoshitake, Ikeda-shi, Osaka 563 (JP); YAMAMOTO, Kazumori, Kawasaki-shi, Kanagawa 211 (JP); TOMII, Akiko, Itami-shi, Hyogo 664 (JP); HARUKAZE, Shinji, Mukou-shi, Kyoto 617 (JP); MATSUMOTO, Kyoichi, Higashiosaka-shi, Osaka 577 (JP); SAMORI, Tomohiro, Amagasaki-shi, Hyogo 660 (JP)
(74) Representative: Patentanwälte Bartels, Held und Partner
(86) International application number: JP9401789
(87) International publication number: WO9525794

(57) **Abstract**

An IGF-I-specific monoclonal antibody and an IGF-I assaying method utilizing the same without using any radioisotope. With respect to ELISA, a monoclonal antibody that does not react with IGF-II, insulin and proinsulin and reacts specifically with IGF-I; and an immunochemical method of assaying IGF-I by using the antibody. The antibody is useful as a reagent for the immunohistochemical study of IGF-I. The ELISA method using the same is a IGF-I assaying method having a clinically practicable sensitivity. Since no radioisotope is used in the assay, this method is effective in simplifying the IGF-I assay.

## Description

### TECHNICAL FIELD

The present invention relates to monoclonal antibodies, and more specifically it relates to monoclonal antibodies which in the immunoresponse system reacts specifically with the C-chain of insulin-like growth factor-I but does not react with insulin-like growth factor-II, insulin or proinsulin.

### BACKGROUND ART

Insulin-like growth factor-I (hereafter sometimes abbreviated to IGF-I) is a polypeptide consisting of 70 amino acid residues, and its amino acid sequence is already known. This substance exhibits insulin-like activity, growth promotion activity, and various other physiological activities acting on the nervous system, kidneys, digestive organs, erythrocytes, thyroid gland, adrenal body, lac glands, muscles and on the vascular system. It is also known that blood concentration of IGF-I is abnormal in cases of acromegaly or hypopituitarism, various diseases resulting in small body height, subnutritional conditions, hepatopathy, hypothyroidism, diabetes, and the like ("Igaku no Ayumi", Vol. 165, No. 5, pp. 264 - 268, 1993). Furthermore, some cases of insulin-resistant diabetes have been found in which blood insulin concentration rises but blood concentration of IGF-I is lowered (ibid., pp. 350 - 353), and these are receiving special clinical attention. Consequently a number of different methods for measuring blood IGF-I concentrations have been developed, and presently the radioimmunoassay technique (Zapp, et al., Journal of Clinical Investigation Vol. 68, pp. 1321 - 1330, 1981) is being widely used.

### DISCLOSURE OF THE INVENTION

Nevertheless, because this assay method uses radioactive isotopes and special equipment is required for their handling, the development of a more simple assay method has been sought. Furthermore, since IGF-I is homologous with proinsulin, insulin and insulin-like growth factor-II (hereunder sometimes abbreviated to IGF-II), the development of a monoclonal antibody has been desired which exhibits no cross-reactivity with them.

The present invention, therefore, provides monoclonal antibodies (I) which, in immunoassay methods, react specifically with the C-chain of insulin-like growth factor-I but do not substantially react with insulin-like growth factor-II, insulin or proinsulin.

The present invention further provides monoclonal antibodies (II) which react with an epitope other than the C-chain of insulin-like growth factor-I and an epitope in common with IGF-II.

The present invention additionally provides hybridoma cell lines which produce the above-mentioned monoclonal antibodies.

The present invention also provides an immunochemical assay method for insulin-like growth factor-I which comprises at least the following steps of:
(1) coating insulin-like growth factor-I on a solid-phase surface which is to be used for the immunoreaction;
(2) pre-reacting a test sample with a monoclonal antibody (I) as a first antibody;
(3) adding to the system the test sample pre-reacted with the first antibody and allowing the unreacted first antibody to react with the insulin-like growth factor-I immobilized on the solid-phase surface;
(4) reacting the first antibody immobilized via the insulin-like growth factor-I immobilized on the solid-phase surface, with a second antibody which is a biotin-labelled antibody specific to the class or subclass of the first antibody;
(5) reacting the biotin portion of the second antibody immobilized via the first antibody and the insulin-like growth factor-I immobilized on the solid-phase surface, with a labelled enzyme which is avidin or streptoavidin labelled with alkaline phosphatase or peroxidase;
(6) adding a substrate to the immobilized labelled enzyme for enzyme reaction to measure the amount of labelling; and
(7) measuring the concentration of insulin-like growth factor-I in the test sample by comparing the labelling count of the test sample with a standard curve for previously measured insulin-like growth factor-I.

The present invention also provides an immunochemical assay method for insulin-like growth factor-I which comprises at least the following steps of:
(1) coating a monoclonal antibody (I) as a first antibody on a solid-phase surface;
(2) adding a test sample to the system and reacting the immobilized first antibody with insulin-like growth factor-I in the sample;
(3) reacting the insulin-like growth factor-I immobilized via the first antibody immobilized on the solid-phase surface, with a second antibody which is a monoclonal antibody (II);
(4) reacting the second antibody immobilized via the insulin-like growth factor-I and the first antibody immobilized on the solid-phase surface, with a third antibody which is biotin-labelled anti-mouse IgG subclass antibody specific only to the second antibody;
(5) reacting the biotin portion of the third antibody immobilized via the second antibody, the insulin-like growth factor-I and the first antibody immobilized on the solid-phase surface, with a labelled enzyme which is avidin or streptoavidin labelled with alkaline phosphatase or peroxidase;
(6) adding a substrate to the immobilized labelled enzyme for enzyme reaction to measure the amount of labelling; and
(7) measuring the concentration of insulin-like growth factor-I in the test sample by comparing the labelling count of the test sample with a standard curve for previously measured insulin-like growth factor-I.

The present invention also provides an immunochemical assay method for insulin-like growth factor-I which comprises at least the following steps of:
(1) coating a monoclonal antibody (II) as a first antibody on a solid-phase surface;
(2) adding a test sample to the system and reacting the immobilized first antibody with insulin-like growth factor-I in the sample;
(3) reacting the insulin-like growth factor-I immobilized via the first antibody immobilized on the solid-phase surface, with a second antibody which is a monoclonal antibody (I);
(4) reacting the second antibody immobilized via the insulin-like growth factor-I and the first antibody immobilized on the solid-phase surface, with a third antibody which is biotin-labelled anti-mouse IgG subclass antibody specific only to the second antibody;
(5) reacting the biotin portion of the third antibody immobilized via the second antibody, the insulin-like growth factor-I and the first antibody immobilized on the solid-phase surface, with a labelled enzyme which is avidin or streptoavidin labelled with alkaline phosphatase or peroxidase;
(6) adding a substrate to the immobilized labelled enzyme for enzyme reaction to measure the amount of labelling; and
(7) measuring the concentration of insulin-like growth factor-I in the test sample by comparing the labelling count of the test sample with a standard curve for previously measured insulin-like growth factor-I.

The present invention also provides an immunochemical assay method for insulin-like growth factor-I which comprises at least the following steps of:
(1) coating a monoclonal antibody (I) as a first antibody on a solid-phase surface;
(2) adding a test sample to the system and reacting the immobilized first antibody with insulin-like growth factor-I in the sample;
(3) reacting the insulin-like growth factor-I immobilized via the first antibody immobilized on the solid-phase surface, with a second antibody which is a monoclonal antibody (II) which has been biotin-labelled;
(4) reacting the biotin portion of the second antibody immobilized via the insulin-like growth factor-I and the first antibody immobilized on the solid-phase surface, with a labelled enzyme which is avidin or streptoavidin labelled with alkaline phosphatase or peroxidase;
(5) adding a substrate to the immobilized labelled enzyme for enzyme reaction to measure the amount of labelling; and
(6) measuring the concentration of insulin-like growth factor-I in the test sample by comparing the labelling count of the test sample with a standard curve for previously measured insulin-like growth factor-I.

The present invention also provides an immunochemical assay method for insulin-like growth factor-I which comprises at least the following steps of:
(1) coating a monoclonal antibody (II) as a first antibody on a solid-phase surface;
(2) adding a test sample to the system and reacting the immobilized first antibody with insulin-like growth factor-I in the sample;
(3) reacting the insulin-like growth factor-I immobilized via the first antibody immobilized on the solid-phase surface, with a second antibody which is a monoclonal antibody (I) which has been biotin-labelled;
(4) reacting the biotin portion of the second antibody immobilized via the insulin-like growth factor-I and the first antibody immobilized on the solid-phase surface, with a labelled enzyme which is avidin or streptoavidin labelled with alkaline phosphatase or peroxidase;
(5) adding a substrate to the immobilized labelled enzyme for enzyme reaction to measure the amount of labelling; and
(6) measuring the concentration of insulin-like growth factor-I in the test sample by comparing the labelling count of the test sample with a standard curve for previously measured insulin-like growth factor-I.

The present invention further provides a kit for immunochemical assay of insulin-like growth factor-I, which comprises 2 types of monoclonal antibodies, namely a monoclonal antibody (I) and a monoclonal antibody (II).

The present invention further provides a kit for immunochemical assay of insulin-like growth factor-I, which comprises 2 types of monoclonal antibodies, namely a monoclonal antibody (I) and a monoclonal antibody (II), one of which has been biotinated.

### BRIEF DESCRIPTION OF THE DRAWINGS

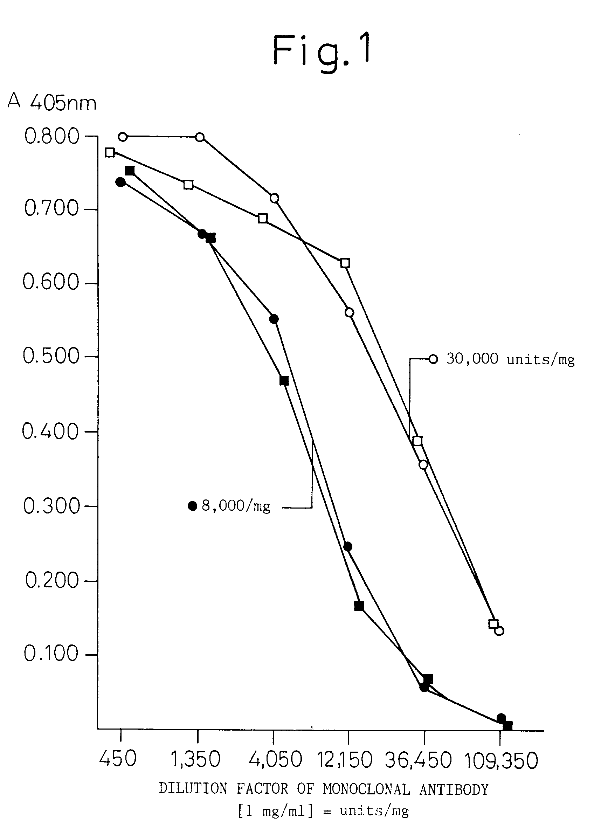

Fig. 1 is a graph showing the antibody titers for antibody 213D9 and antibody 17B2 in Evaluation Example 4. The X-axis represents the dilution factor of the antibody used, and the Y-axis represents the absorbance at 405 nm. In Fig. 1, the unfilled circles represent data where the first antibody was antibody 213D9 and the second antibody was biotinated goat anti-mouse IgG2b antibody, the unfilled squares represent data where the first antibody was antibody 213D9 and the second antibody was biotinated sheep anti-mouse Ig antibody, the solid circles represent data where the first antibody was antibody 17B2 and the second antibody was biotinated goat anti-mouse IgG1 antibody, and the solid squares represent data where the first antibody was antibody 213D9 and the second antibody was biotinated sheep anti-mouse Ig antibody.

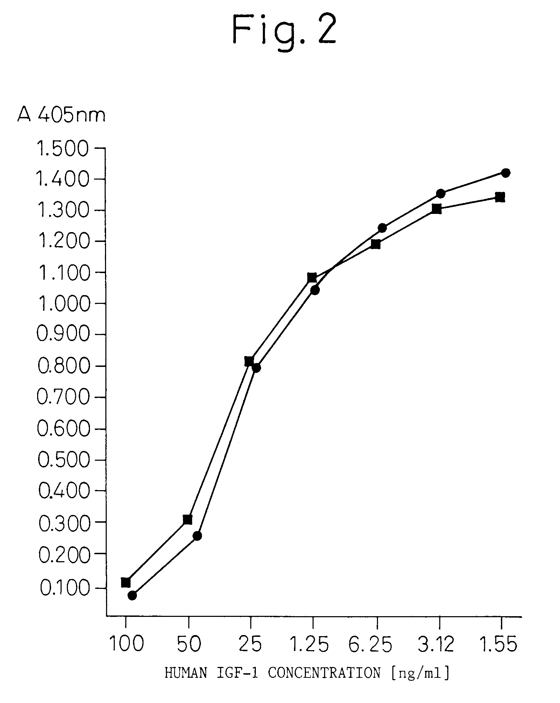

Fig. 2 shows a standard curve for IGF-I assay using antibody 17B2 in Example 4. The X-axis represents IGF-I concentration (ng/ml) in the sample and the Y-axis represents absorbance at 405 nm. In Fig. 2, the black circles represent data where the first antibody was antibody 17B2 and the second antibody was biotinated goat anti-mouse IgG1 antibody, and the black squares represent data where the first antibody was antibody 213D9 and the second antibody was biotinated sheep anti-mouse Ig antibody.

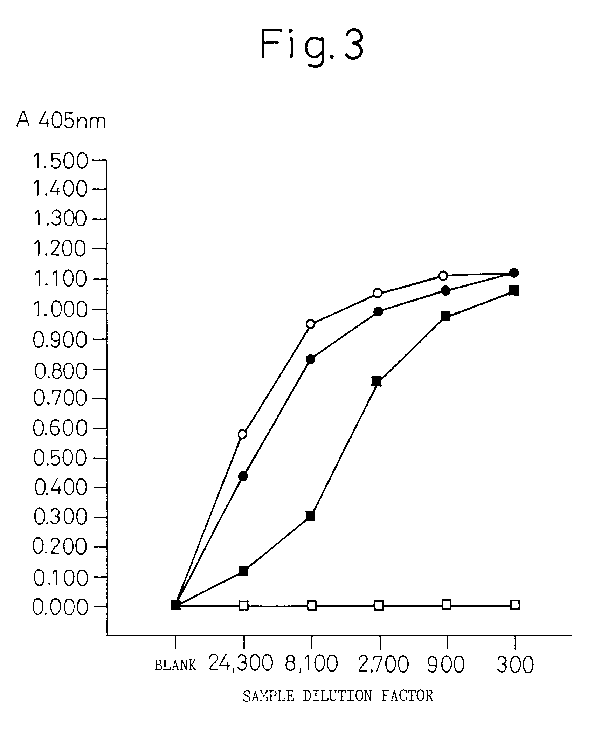

Fig. 3 is a graph drawn to examine the reactivities of the antibodies in Evaluation Example 5. The X-axis represents dilution factor of the added antibody, and the Y-axis represents the absorbance at 405 nm. In Fig. 3, the black circles represent data where antibody 27E10 was reacted with IGF-I, the black squares represent data where antibody 27E10 was reacted with the IGF-I-C fragment [30-41], the white circles represent data where antibody 16G4 was reacted with IGF-I and the white squares represent data where antibody 16G4 was reacted with the IGF-I-C fragment [30-41].

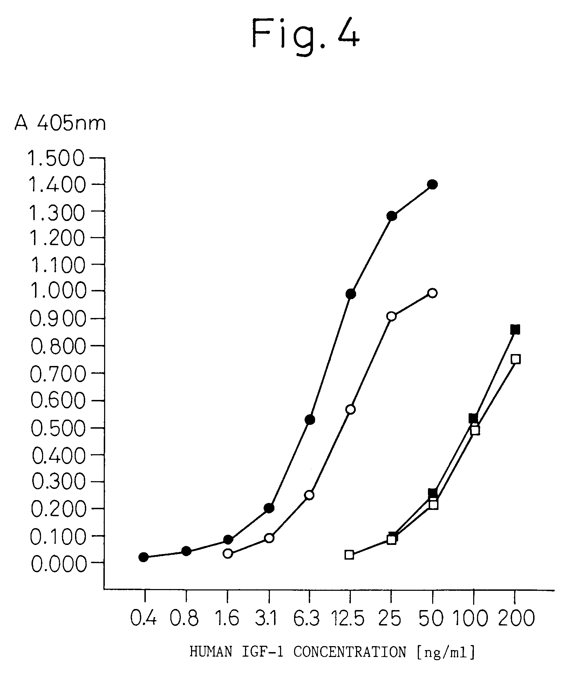

Fig. 4 shows a standard curve for IGF-I assay by the double antibody method in Example 6. The X-axis represents IGF-I concentration (ng/ml) in the sample and the Y-axis represents absorbance at 405 nm. In Fig. 4, the black circles represent data where the first antibody was antibody 27E10 and the second antibody was biotinated 16G4 antibody, the black squares represent data where the first antibody was antibody 213D9 and the second antibody was biotinated 16G4 antibody, the white circles represent data where the first antibody was antibody 27E10 and the second antibody was biotinated 22G4 antibody, and the white squares represent data where the first antibody was antibody 213D9 and the second antibody was biotinated 22G4 antibody.

### DETAILED DESCRIPTION

The present inventors concentrated on ELISA (Enzyme-Linked Immunosorbent Assay) as a non-radioisotope assay method, and as a result of diligent research aimed at obtaining a hybridoma producing monoclonal antibody with sufficient titer in ELISA, succeeded in obtaining a number of such hybridomas, thus completing the present invention. In other words, hybridoma lines were prepared by immunizing mice with the immunogens IGF-I-bound bovine serum albumin (hereunder, BSA-IGF-I) and IGF-I-bound ovalbumin (hereunder, OVA-IGF-I), and fusing spleen cells from the sensitized mice with a myeloma cell line (P₃-X63-Ag₈-U₁) (hereunder sometimes abbreviated to P₃U₁). The antibody titers of the culture supernatants of these hybridomas were measured by the ELISA method and hybridomas exhibiting high titers were selected. The obtained hybridomas were then intraabdominally cultured in mice, and the ascitic supernatants were purified by ammonium sulfate precipitation, Sephadex G-25M (product of Pharmacia) and protein G-Sepharose (product of Pharmacia) to obtain monoclonal antibodies. These monoclonal antibodies exhibited a high titer, making them suitable for IGF-I assay by the ELISA method.

According to the present invention, monoclonal antibodies may be obtained in the following manner. That is, first BALB/c mice, etc. are immunized with an immunogen. The immunogen may be the antigen IGF-I itself, but for higher immunogenicity it is preferably bound to a high molecular carrier such as, for example, bovine serum albumin, ovalbumin, keyhole limpet hemocyanin (KLH), thyroglobulin or a metal colloid (for example, gold or iron colloid) prior to immunization. For higher antibody productivity, the immunization is preferably carried out using a water-in-oil (w/o) emulsion with an appropriate adjuvant [for example, Freund's complete adjuvant (FCA) for the initial immunization and Freund's incomplete adjuvant (FICA) for booster immunizations], with 1 to 5 (preferably 3 to 4) booster immunizations after the initial immunization.

Between the 7th and 10th days after the final immunization, blood samples are taken from the eyeground venous plexus, the serum antibody titer is measured by the ELISA method, and sensitized mice are selected. The selected mice are injected with an aqueous solution of the antigen via the caudal vein 3 days prior to the cell fusion. Just prior to the cell fusion, the mice are bled and the blood collected, and serum is separated and cryopreserved as control serum for the assay. The bled mice are immediately cut open at the abdomen, the spleens are aseptically extracted, and hybridomas are prepared according to a conventional method (Keller, et al., Nature, Vol. 256, pp. 495 - 497, 1975).

Next, the obtained hybridomas are selectively cultured using HAT medium, the IGF-I-specific antibody titers of the supernatants are measured by the ELISA method, and high-titers hybridomas are selected. Clones are then selected by limiting dilution or by colony formation in soft agar, the culture supernatants of the hybridomas are tested for immunological cross-reactivity with insulin, proinsulin and IGF-II in addition to IGF-I, and selection is made of those which recognize IGF-I alone or IGF-I and IGF-II but do not cross-react with the others. The hybridoma clones obtained in this manner are cultured, and the antibody titers of the supernatants are measured by the ELISA method while the antibody class, subclass and type of each is also determined by the ELISA method.

A monoclonal antibody may be obtained by using a conventional method to purify antibody from the ascitic supernatant obtained by culturing the hybridoma clones obtained above in the abdomen of mice intraperitoneally injected beforehand with an ascites-inducing agent. The obtained monoclonal antibody may be used for assay of IGF-I by the ELISA method in the conventional manner. A kit for ELISA assay of IGF-I may also be prepared by combining, in addition to the monoclonal antibody, common reagents used for ELISA, such as biotinated goat anti-mouse Ig antibody, alkaline phosphatase-labelled avidin or likewise-labelled streptoavidin and the substrate disodium p-nitrophenylphosphate, peroxidase-labelled avidin or likewise-labelled streptoavidin and substrates such as 4-chloro-1-naphthol, 3-amino-9-ethylcarbazole (AEC) and diaminobentidine (DAB), as well as coating solutions used for antigen coating, blocking solutions used after coating, washing solutions used between each reaction, buffering solutions used for each reaction, a 96-well plate for ELISA, etc.

Of the 8 hybridomas obtained in this manner, including 17B2, 213D9, 27E10, etc., hybridoma 17B2 (named hybridoma 17B-2) was deposited at the National Institute of Bioscience and Human Technology on February 17, 1994 as FERM BP-4572, and the antibody it produces is subclass IgG1-K. Also, hybridoma 213D9 (named hybridoma 213D-9) was deposited at the National Institute of Bioscience and Human Technology on the same date as FERM BP-4570, and the antibody it produces is subclass IgG2b-K. Hybridoma 27E10 (named hybridoma 27E-10) was deposited at the National Institute of Bioscience and Human Technology on October 7, 1994 as FERM BP-4827, and the antibody it produces is subclass IgG1-K.

The present inventors also obtained 25 other hybridomas, including 16G4, 22G4, 25D4, etc. producing monoclonal antibodies which recognized common epitopes of the molecular structure of IGF-I and IGF-II. Of these, the antibodies produced by 16G4 and 22G4 are both subclass IgG1-K, and the antibody produced by 25D4 is subclass IgG2a-K. Of these hybridomas, hybridoma 25D4 (named hybridoma 25D-4) was deposited at the National Institute of Bioscience and Human Technology on February 17, 1994 as FERM BP-4571 and hybridoma 16G4 (named hybridoma 16G-4) was deposited at the National Institute of Bioscience and Human Technology on October 7, 1994 as FERM BP-4826.

The monoclonal antibodies produced by hybridoma 17B2, hybridoma 27E10 and hybridoma 213D9 according to the present invention (hereunder, the monoclonal antibodies produced by these hybridomas will be referred to by the numbers of the hybridomas, i.e. antibody 17B2, antibody 27E10 and antibody 213D9) react solely and specifically with IGF-I and do not cross-react with insulin, proinsulin or IGF-II, and their detectability with ELISA is comparable to the RIA method. Furthermore, although antibodies 25D4 and 22G4 cross-react with IGF-II, their subtypes and epitopes differ from the previously mentioned antibodies, and thus they may be used together with antibody 17B2 or antibody 213D9 in the double antibody method (sandwich method) for even greater detectability. Thus, according to the present invention, IGF-I may be specifically assayed without using radioactive isotopes.

### EXAMPLES

The present invention will now be explained in greater detail by way of the following examples which, however, in no way limit the scope of the present invention.

### Example 1: Preparation of immunogen

### a. Preparation of bovine serum albumin-bound IGF-I (BSA-IGF-I)

hIGF-I, with a low molecular weight of 7,649, has low antigenicity and thus hIGF-I itself is believed to be a hapten antigen; in order to increase its immunogenicity, therefore, it was bound to appropriate carrier proteins to prepare complexes with higher molecular weight which were thought to be suitable as immunogens. The carrier proteins selected were bovine serum albumin (BSA) and ovalbumin (OVA), and the respective protein-IGF-I complexes were prepared.

The preparation of BSA-IGF-I was conducted according to the DCC method. That is, 2.5 mg of bovine serum albumin (BSA, Sigma, code A-7511) was dissolved in 400 µl of distilled water, and then 5 mg of 1-ethyl-3-(3-dimethylaminopropyl)carbodiimide·HCl (WSDCC, Nakaraitesk, code 150-22) dissolved in 100 µl of distilled water was added thereto while stirring, and the mixture was allowed to stand at 37°C for one hour. Following this, 0.8 mg of gene recombinant human IGF-I (rh-IGF-I, Fujisawa Yakuhin, code FK780) dissolved in 1.2 ml of distilled water was added while stirring, and the mixture was allowed to stand at 37°C for one hour. After completion of the reaction, the protein fractions were collected with a Sephadex G-25 mini-column for protein purification (PD10, product of Pharmacia) and lyophilized to prepare BSA-IGF-I.

### b. Preparation of ovalbumin-bound IGF-I (OVA-IGF-I)

The preparation of OVA-IGF-I was also conducted according to the DCC method in the same manner as BSA-IGF-I. That is, 2.5 mg of ovalbumin (OVA, Seikagaku Kogyo code: 250440) was dissolved in 400 µl of distilled water, 6.8 mg of WSDCC dissolved in 100 µl of distilled water was added thereto while stirring, and the mixture was allowed to stand at 37°C for one hour. Following this, 0.8 mg of rh-IGF-I dissolved in 1.2 ml of distilled water was added while stirring, and the mixture was allowed to stand at 37°C for one hour. After completion of the reaction, the protein fractions were collected with a Sephadex G-25 mini-column for protein purification and lyophilized to prepare OVA-IGF-I.

### Example 2: Preparation of hybridomas

### a. Sensitization of mice with BSA-IGF-I

The lyophilized BSA-IGF-I prepared in Example 1 was dissolved in Dulbecco PBS(-) to pH 7.4 [Nissui, code 05913, hereafter sometimes abbreviated to D'-PBS(-)] to prepare a 1 mg/ml antigen solution. Freund's complete adjuvant (FCA, Calbiochem-Behring, code 250440) and the above-mentioned antigen solution were mixed in a ratio of 3:2 to prepare a w/o emulsion. This emulsion was subcutaneously injected into 8 BALB/c mice (female, SPF grade, 26-29 days old, purchased from Nihon Crea) at a dose of 125 µg of antigen per mouse for initial immunization. After 2 weeks, Freund's incomplete adjuvant (FICA, Difco, code 0639-60-6) and the above-mentioned antigen solution were mixed in a ratio of 3:2 to prepare a w/o emulsion which was injected into the abdomen of the mice at a dose of 63 µg of antigen per mouse, as a booster immunization. They were given additional booster immunizations of 31 µg/mouse twice at one week intervals (4 times including the initial immunization). At one week after the final immunization, blood samples were taken from the eyeground venous plexus of the sensitized mice and the titer of serum antibody against OVA-IGF-I, with a different type of carrier protein constructing the immunogen, was measured by the ELISA method described below as an indication of antibody production. Since antibody production was confirmed in all of the sensitized mice, 4 of the mice providing cells for cell fusion were intravenously injected with 37 µg/mouse of antigen dissolved in D'-PBS(-), and on the 3rd day thereafter the mice were bled and slaughtered and their spleens were extracted to be used for the first hybridoma preparation.

The remaining 4 mice were given a 5th booster immunization 10 days after the 4th injection, blood samples were taken from the eyeground venous plexus in the same manner 2 weeks after the final immunization, and the titer of serum antibody against OVA-IGF-I was measured in the same manner as an indication of antibody production. Since antibody production was confirmed in all 4 of the mice, the mice were intravenously injected with 31 µg/mouse of antigen dissolved in D'-PBS(-), and on the 3rd day thereafter the mice were bled and slaughtered and their spleens were extracted to be used for the second hybridoma preparation.

### b. Sensitization of mice with ovalbumin-bound antigen

The lyophilized OVA-IGF-I prepared in Example 1 was dissolved in D'-PBS(-) to prepare a 1 mg/ml antigen solution. In the same manner as step (a.) above, the titer of serum antibody against BSA-IGF-I, with a different carrier protein constructing the immunogen, was used as the indicator for hybridoma preparation carried out twice for each of the 4 mice.

### c. Hybridoma preparation

The sensitized mice obtained by the immunization in (a.) and (b.) above were bled, the blood was collected, and the serum was separated and cryopreserved as control serum for the assay. The bled mice were immediately cut open at the abdomen, and the spleens were aseptically extracted and loaded into a receptacle containing RPMI-140 medium (Nissui, code 05918) adjusted to pH 7.4 with a 7.5% sodium bicarbonate solution. The rest of the procedure was performed in a clean bench. After washing the extracted spleens twice in RPMI-1640 medium, the adhering fat tissue was cut off and scissors were used to slice the spleens into 2 longitudinal sections which were broken up in the medium with forceps. The resulting cell suspension was filtered and collected with a stainless steel wire net sterilized with an autoclave. This was centrifuged at low speed and the supernatant was removed to obtain a deposited packed cell pellet. The erythrocytes were removed from the pellet by the Ficoll-Hypaque method, and after washing once with RPMI-1640 medium the cells were resuspended in the same medium and a cell count was taken.

Meanwhile, fresh myeloma cells (P₃U₁) which had been rapidly grown for 2-3 days in GIT medium (Nihon Seiyaku, code 398-00515) were centrifuged at low speed (1000 rpm, 3 minutes) using a round-bottomed centrifuge tube, the supernatant was discarded, the deposited packed cell pellet was broken up by tapping, and after washing twice with RPMI-1640 medium (pH 7.4) heated to 37°C, the cells were resuspended in the same medium and a cell count was taken.

The resulting pellet cell suspension and fresh cell suspension (P₃U₁) were dispensed into round-bottomed tubes for cell fusion at a calculated cell count proportion of about 5:1, and after low-speed centrifugation and removal of the supernatant, the deposited packed cell pellets were broken up by tapping. Polyethylene glycol (PEG), which had been sterilized by autoclave and then cooled to about 50°C, was dissolved in an equivalent amount of RPMI-1640 medium, and then 2 ml of the solution adjusted to pH 7.4 with 7.5% sodium carbonate was slowly dispensed into the tubes and mixed with the contents over 20-30 seconds while keeping the temperature at 37°C. An 8 ml portion of RMPI-1640 medium at 37°C was then gradually added thereto while slowly mixing with a pipette.

This was then centrifuged at low speed (800 rpm, 3 minutes) at 25-37°C and the supernatant was discarded, the cell pellet was broken up while gradually adding 10 ml of HT medium [RPMI-1640 medium containing hypoxanthine (nacalai tasque, code 190-01 Kojin), thymidine (nacalai tasque, code 338-25 Kojin) and 10% fetal calf serum], and after resuspension and one washing procedure, the cells were resuspended in HT medium to a calculated myeloma cell count of 1 × 10⁶ cells/ml. This was dispensed into a 96-well cell culturing plate at 0.1 ml/well, and culturing was performed in a CO₂ gas incubator (5% CO₂ gas concentration, 37°C).

On the 2nd day of culturing, 50 µl of HAT medium [RPMI-1640 medium containing hypoxanthine, aminopterin (Sigma, code A-1784), thymidine and 10% fetal calf serum] was added to each well. On every 3rd day thereafter, the medium was replaced with 100 µl of HAT medium per well. After 2 weeks, the growth of the hybridoma colonies was observed under a microscope, and the culture supernatants obtained upon medium replacement in those wells confirmed to have hybridoma colonies were subjected to antibody titer measurement by the same method used for the serum antibody titer measurement by ELISA. The cells confirmed to exhibit the desired antibody production had their HAT medium replaced with HT medium to induce cell growth, and then GIT medium was used for scaling up in order from 1 ml/well to 10 ml/T flask.

The thymuses were extracted from female mice (BALB/c, 4 weeks old), and suspended in GIT medium containing hypoxanthine and thymidine, at 5 × 10⁶ cells/ml. Hybridomas obtained using these thymus cells were subjected to limiting dilution to concentrations of 5 cells/0.1 ml/well, 2.5 cells/0.1 ml/well, 1.25 cells/0.1 ml/well and 0.625 cells/0.1 ml/well, culturing was performed for 10 days to 2 weeks in a CO₂ gas incubator (5% CO₂ gas concentration, 37°C), and the cell growth and antibody titers of the supernatants were measured. Recloning when necessary, 45 BSA-IGF-I-reacting clones were obtained from mice immunized with OVA-IGF-I, and 3 of these clones reacting with IGF-I itself were obtained. Of these 3 clones, 2 were found to be specific to IGF-I by the identification of specificity described below. Also, 161 OVA-IGF-I-reacting clones were obtained from mice immunized with BSA-IGF-I, and 58 of these clones reacting with IGF-I itself were obtained. Of these clones, 6 were found to be specific to IGF-I by the identification of specificity described below. That is, a total of 61 IGF-I-reacting clones were obtained, 8 of which were IGF-I-specific clones.

After subculturing, the obtained hybridomas were centrifuged at low speed (1000 rpm, 15 - 25°C, 3 minutes), the supernatants were removed, and then the deposited packed cell pellets were broken up by tapping. RPMI-1640 medium containing a solution cryopreserved at 4°C (10% dimethylsulfoxide, 20% fetal calf serum) was added thereto to prepare a cell suspension with 5 × 10⁶ cells/ml. This was dispensed into 1.8 ml cryopreservation tubes at 1 ml per tube, and after prefreezing at -80°C overnight, the tubes were stored in liquid nitrogen (-194°C).

### Evaluation Example 1: Antibody assay by ELISA

### a. Preparation of antigen-coated plate

Each of the antigens to be assayed (using OVA-IGF-I in the culture supernatants of clones sensitized with BSA-IGF-I and using BSA-IGF-I in those sensitized with OVA-IGF-I) was dissolved in distilled water to prepare a 1 mg/ml aqueous solution which was stored at -80°C. At the time of use, a coating buffer solution (0.1 M carbonate/bicarbonate buffer solution, pH 9.6) was used for dilution to a concentration of 10 µg/ml, 100 µl thereof was dispensed into each well of a 96-well microtiter plate for EIA (Costar, code 3590), and the plate was allowed to stand at room temperature for 2 hours. The antigen solution was drawn out from each well, and the plate was washed 3 times with a washing solution [D'-PBS(-) containing 0.1 w/v% Tween 20 (Biorad, code 170-6531) and 0.1 w/v% NaN₃]. Next, 150 µl of a blocking solution [D'-PBS(-) containing 0.1 w/v% gelatin (Biorad, code 170-6537) and 0.1 w/v% NaN₃] was added to each well, and incubation was performed at 37°C for 30 minutes. After removing the blocking solution from each well, the plate was washed 3 times with the washing solution to prepare an antigen-coated plate.

### b. Antigen-antibody reaction

A sample to be tested (first antibody) was diluted and 100 µl thereof was added to each well and incubated at 37°C for one hour to allow reaction with the coating antigen. After removing the sample, the plate was washed 3 times with the washing solution. A second antibody solution (Amersham, code RPN1009, 500-fold dilution of sheep biotinated anti-mouse Ig antibody) was then added thereto at 100 µl/well and incubation was performed at 37°C for one hour. After removing the supernatant, the plate was washed 3 times with the washing solution. This procedure resulted in immobilizing the second antibody in an amount corresponding to the activity of the first antibody in the sample.

### c. Biotin-avidin reaction

An alkaline phosphatase-labelled avidin solution (Dacopatz, code D365, 500-fold dilution) was added at 100 µl/well, and incubation was performed at 37°C for 30 minutes. After removing the supernatant, the plate was washed 3 times with the washing solution. This procedure resulted in immobilizing the alkaline phosphatase in an amount corresponding to the activity of the first antibody in the sample.

### d. Alkaline phosphatase reaction

A substrate solution [disodium p-nitrophenylphosphate (PNPP, Wako Junyaku, code 149-02342) dissolved to a concentration of 1 mg/ml in a 10 mM diethanolamine buffer solution (pH 9.5) containing 0.5 mM MgCl₂] was added at 100 µl/well and the plate was allowed to stand at room temperature for 20 minutes, after which a reaction-suspending solution (3N NaOH) was added at 50 µl/well to suspend the enzyme reaction, and an automatic absorbance meter was used to measure the absorbance at 405 nm. The enzyme reaction-positive samples were judged to be antibody-positive, and the dilution factor of each sample corresponding to a mid-point of the linear concentration gradient of the absorbance sigmoid curve was used as an index of the antibody titer (U/ml).

### Evaluation Example 2: Identification of monoclonal antibody subclasses

The subclasses of the obtained monoclonal antibodies were identified by ELISA. That is, culture supernatants of the obtained hybridomas cultured in GIT medium were added to a co-binding plate (Micromembrane, code CB-PL-1) at 100 µl/well and incubated at 37°C for one hour to bind the antibodies to the plate. A blocking solution was then added at 150 µl/well and incubation was continued at 37°C for one hour, after which each solution was removed and the plate was washed 3 times with washing solution. Antibody solutions [500-fold dilutions of goat-derived biotinated antibody specific to each class, subclass and type of mouse immunoglobulin {anti-IgG1 (code RPN1180), anti-IgG2a (RPN1181), anti-IgG2b (RPN1182), anti-IgG3 (RPN1183), anti-IgA (RPN1175), anti-IgM (RPN1176), anti-K chain (RPN1179), anti-γ chain (RPN1178), all products of Amersham}] were added to each plate at 100 µl/well and incubated at 37°C for one hour, the solutions were removed, and then the plates were washed 3 times with washing solution.

Then, in the same manner as in (c) and (d) of Evaluation Example 1, alkaline phosphatase-labelled avidin solution addition, incubation, washing, substrate solution addition and reaction-suspending solution addition were followed by measurement of the absorbance at 405 nm using an automatic absorbance meter in the same manner. The class, subclass and type of each of the antibody solutions added to the enzyme reaction-positive wells were identified to determine the class, subclass and type of the monoclonal antibodies.

### Evaluation Example 3: Judgment of monoclonal antibody specificity

The ELISA method was used to determine the specificity of culture supernatants of the 61 IGF-I-reacting hybridomas obtained in Example 2, with respect to IGF-I, IGF-II, proinsulin and insulin. That is, 2 types of gene recombinant human IGF-I (Fujisawa Yakuhin Kogyo, code FK780 and Amersham, code ARM4050), gene recombinant human IGF-II (Amersham, code ARM24050), gene recombinant human proinsulin (Sigma, code P4672) and gene recombinant human insulin (Sigma, code I02959) as antigens were each dissolved in a 0.1 M sodium carbonate buffer solution (pH 9.6) to a concentration of 8 µg/ml, 80 µl thereof was added to wells of a 96-well EIA plate (Costar, code 3590), and the plate was allowed to stand at 37°C for one hour. After blocking and washing in the same manner as in Evaluation Example 1, a sample to be tested (first antibody, hybridoma culture supernatant) was diluted, added at 100 µl/well and incubated at 37°C for one hour, the sample was removed, and then the plate was washed 3 times with washing solution.

The rest of the treatment was conducted in the same manner as in (b), (c) and (d) of Evaluation Example 1. That is, addition of the second antibody solution and incubation were followed by removal of the supernatant and washing. Addition of alkaline phosphatase-labelled avidin solution and further incubation were followed by removal of the supernatant and washing. Then, after addition of the substrate solution and allowing the plate to stand at room temperature for 20 minutes, reaction-suspending solution (3 N NaOH) was added at 50 µl/well to suspend the enzyme reaction, and an automatic absorbance meter was used to measure the absorbance at 405 nm. Enzyme reaction-positivity was judged as antibody-positivity.

Table 1 shows the reactivities of the antibodies produced by the 61 IGF-I-reacting clones with respect to IGF-II, insulin and proinsulin.

**Table 1**

| Reactivities of Obtained Antibodies | | |
|---|---|---|
| Reactivity | Number of clones | Name of clone |
| Anti-IGF-I specific | 8 | 13B2, 213D9, 17B2, 21D3, 25C8, 25F2, 27E10, 29G6. |
| Anti-IGF-I + II | 25 | 12E10, 15D4, 16G4, 21D2, 21F7, 22C3, 22D7, 22F4, 22G4, 23D9, 23E6, 24D5, 25B6, 25D4, 27E9, 28C8, 28C10, 29C2, 29D4, 29F6, 29F7, 29F8, 210C2, 210D2, 210D2-2. |
| Anti-IGF-I + II + In | 8 | 21C7, 21G5, 22F3, 23F11, 23G6, 24E8, 25C7, 210E11. |
| Anti-IGF-I + II + In + Pr | 19 | 21C9, 25B7, 25D5, 26E3, 26F9, 26F11, 27B5, 27C2, 27C11, 27D10, 28D8, 28E6, 28F5, 29B6, 210B6, 210B7, 210E9, 210F10, 210G6. |
| Anti-IGF-I + In + Pr | 1 | 28F11. |
| Abbreviations: In = insulin, Pr = proinsulin | | |

### Example 3: Production of monoclonal antibodies by invivo culturing

Hybridoma 17B-2 and hybridoma 213D-9 were cultured in GIT medium and then washed with RPMI-1640 basic medium to prepare cell suspensions with 10⁷ cells/ml. Female BALB/c mice between 5 and 6 weeks of age were intraperitoneally injected twice with the ascites-inducing agent pristane (2,6,10,14-tetramethylpentadecane, Aldrich, code T2280-2) at 0.5 ml/mouse, and after about 2 weeks the above-mentioned hybridoma cell suspensions were intraperitoneally injected at 0.5 ml/mouse (5 × 10⁶ cells/mouse). The ascites fluid pooled within 1 to 10 days, and upon enlargement of the abdomen the ascites fluid was taken with a syringe. In this manner, 5 - 10 ml of ascites fluid was obtained from each mouse.

The obtained ascites fluid was centrifuged at 4°C, 3000 rpm for 10 minutes and the supernatant was collected, and then an equivolume of a saturated ammonium sulfate aqueous solution (pH 7.4) was added to the ascites supernatant for ammonium sulfate precipitation (50% saturation). After the precipitation was complete, centrifugation was conducted at 4°C, 10,000 x g for 30 minutes, the supernatant was removed, the precipitated fraction was dissolved in a small amount of physiological saline, and the ammonium sulfate was removed with a column using Sephadex G-25M (product of Pharmacia). After subsequent delipidization with a cooled refrigerant, Freegen (trichlorotrifluoroethane, Behring Res. Lab., code OSVW19605189/Eu), the protein concentration was adjusted to about 5 mg/ml.

Each sample was then diluted with a bonding buffer solution (Affi-Gel Protein G MAPS-II buffer solution, Biorad, code 153-6160, pH 9.0) at a ratio of 1:1, and this was subjected to column chromatography using Protein G-Sepharose (Pharmacia-LKB, code 17-0618-01) which had been washed with a 20-bed volume of bonding buffer solution, and after washing with a 15-bed volume of bonding buffer solution, elution was performed with a 10-bed volume of an eluting buffer solution (having the same composition as the binding buffer solution with the pH adjusted to 3.0) to elute out the IgG fraction. To adjust the pH of the eluate to neutral, 25 ml at a time was fractionated in a receptacle already containing 8 ml of 1 M Tris-HCl buffer solution (pH 9.5). The recovered antibody components were collected, the solvent was replaced with physiological saline using an ultrafiltration membrane (cutoff: 30,000), and the solutions were lyophilized.

### Evaluation Example 4: Quantitation of monoclonal antibody titers

### a. Preparation of antigen plates

Measurement of the titers of each of the purified monoclonal antibodies obtained in Example 3 was made by the ELISA method in the same manner as in Evaluation Example 1. That is, gene recombinant h-IGF-I (Amersham, code ARM4050) was dissolved in a coating buffer solution (0.1 M carbonate buffer solution, pH 9.6) to 1 ug/ml, 100 µl thereof was dispensed into each well of a 96-well EIA plate (Costar, code 3590) and incubated at 37°C for one hour. After drawing and collecting the solution out from each well, the plate was washed 3 times with a washing solution [D'-PBS(-) containing 0.1 w/v% Tween 20 and 0.1 w/v% NaN₃]. Next, 150 µl of a blocking solution [D'-PBS(-) containing 0.1 w/v% gelatin and 0.1 w/v% NaN₃] was added to each well, and incubation was performed at 37°C for 30 minutes. After removing the blocking solution from each well, the plate was washed 3 times with the washing solution to prepare an antigen plate.

### b. Antigen-antibody reaction

Each of the samples obtained in Example 3 was dissolved in blocking solution to a concentration of 1 mg/ml, and this was diluted 450-fold, 1350-fold, 4050-fold, 12,150-fold, 36,450-fold and 109,350-fold. These were dispensed into antigen plates at 10 µl/well for incubation at 37°C for 45 minutes. After removal of the supernatant and washing 3 times, a second antibody solution (500-fold dilution of sheep biotinated anti-mouse Ig antibody, or sheep biotinated anti-mouse IgG1 antibody for 17B2 and sheep biotinated anti-mouse IgG2b antibody for 213D9, all products of Amersham) was then added thereto at 100 µl/well and incubation was performed at 37°C for 45 minutes, after which the supernatant was removed and the plates were washed 3 times with washing solution.

### c. Biotin-avidin reaction

An alkaline phosphatase-labelled avidin solution (Dacopatz, code D365, 500-fold dilution) was added at 100 µl/well, and incubation was performed at 37°C for 30 minutes, after which the supernatant was removed and the plates were washed 3 times with washing solution.

### d. Alkaline phosphatase reaction

A substrate solution [PNPP dissolved to a concentration of 1 mg/ml in a 10 mM diethanolamine buffer solution (pH 9.5) containing 0.5 mM MgCl₂] was added at 100 µl/well and the plates were allowed to stand at room temperature for 30 minutes, after which a reaction-suspending solution (3N NaOH) was added at 50 µl/well to suspend the enzyme reaction, and an automatic absorbance meter was used to measure the absorbance at 405 nm.

### e. Calculation of antibody titers

The data obtained was plotted on semilogarithmic graph with a Y-axis of absorbance and an X-axis (logarithmic scale) of dilution factor of the sample, to prepare a sigmoid curve as shown in Fig. 1.

Since a sigmoid curve is drawn for diluted samples of specific antibody, the titer is shown as the dilution factor corresponding to a mid-point of the linear concentration gradient region of the curve. In Fig. 1, the dilution factor corresponding to a mid-point on the linear concentration gradient region is 8000-fold for antibody 17B2 and 30,000-fold for antibody 213D9, and therefore the titer of antibody 17B2 is 8000 unit/mg and the titer of antibody 213D9 is 30,000 unit/mg.

### Example 4: Quantitation of IGF-I by ELISA

### a. Drawing of standard curve

A standard sample (gene recombinant IGF-I, Amersham, code ARM 4050) was diluted to 100 ng/ml, and this was further diluted by various factors (1, 2, 4, 8, 16 32 and 64-fold) and dispensed in a 96-well V-bottomed plate for sample preparation at 60 µl/well. Antibody 17B2 obtained in Example 3 adjusted to 4 unit/ml was dispensed at 60 µl/well and reacted at 37°C for 30 minutes. The reacted solution was then dispensed at 100 µl/well into an antigen plate prepared in the same manner as in Evaluation Example 3-a and incubated at 37°C for 45 minutes. After removal of the supernatant and washing 3 times, a second antibody solution (500-fold dilution of sheep biotinated anti-mouse Ig antibody, or biotinated anti-mouse IgG1 antibody: all products of Amersham) was then added thereto at 100 µl/well and incubation was performed at 37°C for 45 minutes, after which the supernatant was removed and the plate was washed 3 times with washing solution. The absorbance thereof was measured in the same manner as in Evaluation Example 3-a and 3-d. The data obtained was plotted as a semilogarithmic graph with a Y-axis of absorbance and an X-axis (logarithmic scale) of sample dilution factor, to prepare a sigmoid curve as shown in Fig. 2. This standard curve demonstrates that it is possible to assay IGF-I in a range of about 1.6-50 ng/ml when biotinated anti-mouse IgG1 antibody is used as the second antibody.

### b. Assay of IGF-I in human serum

After addition of 400 µl of a pretreatment agent (25 ml of 12 N hydrochloric acid and 100 ml of water combined with ethanol to a total volume of 1000 ml) to 100 µl of normal adult human pooled serum and stirring for 30 minutes, the mixture was centrifuged at 1,500 x G for 30 minutes, 100 µl of the supernatant was taken, and 600 µl of a neutralizing agent (1.74 g of KH₂PO₄, 15.52 g of Na₂HPO₄·H₂O and 0.5 g of NaN₃ combined with water to a total volume of 1000 ml) was added for neutralization. By this procedure, the protein-binding IGF-I was separated, isolated IGF-I was obtained, and a 35-fold dilution was prepared.

Without further treatment the sample was then diluted 2-fold and 4-fold (for total dilutions of 35-fold, 70-fold and 140-fold) and dispensed into a 96-well V-bottomed plate for sample preparation at 60 µl/well in the same manner as in (a.) above and treated with biotinated anti-mouse IgG1 antibody as the second antibody, and then the absorbance thereof was measured in the same manner as in (a.) above. The absorbance was 1.245 for the original sample, 1.425 for the 2-fold diluted sample and 1.497 for the 4-fold diluted sample. These data were fitted to the data of the graph plotting absorbance in the concentration gradient region, and the corresponding IGF-I concentrations were read and multiplied by the dilution factors to determine the respective serum IGF-I concentrations. Using the data read for the original isolated sample (total dilution: 35-fold) as an example, the assayed value was 6.18 × 35 = 216.65 (ng/ml).

### Example 5: Determination of epitope recognized by monoclonal antibody

The epitope recognized by anti-IGF-I-specific antibody was investigated by the ELISA method, in the same manner as in Evaluation Example 1. That is, a 1 µg/ml rh-IGF-I solution (Fujisawa Yakuhin) or 5 µg/ml IGF-I C-domain [30-41] fragment solution (Bachem) dissolved in a coating buffer solution was dispensed into each well of an 96-well microtiter for EIA (Costar) at 100 µl/well, to coat the wells. Washing and blocking were then performed in the same manner as in Evaluation Example 1.

Antibody 27E10 and antibody 16G4 solutions were then adjusted to concentrations of 1 mg/ml and diluted to 300-fold, 900-fold, 2,700-fold, 8,100-fold and 24,300-fold, and each was added at 100 µl/well for reaction. After completion of the immunoreaction, they were labelled and reacted with an enzyme in the same manner as in Evaluation Example 1 and the absorbance at 405 nm was measured.

The results are shown in Fig. 3. As shown here, when IGF-I was immobilized on a solid phase and reacted with the IGF-I-specific antibody 27E10 (black circles), the IGF-I reacted with antibody 27E10 in a concentration-dependent manner. When the IGF-I [30-41] fragment corresponding to the C-domain was immobilized on a solid phase (black squares), the IGF-I [30-41] fragment differed in sensitivity but still clearly reacted with antibody 27E10 in a concentration-dependent manner. In contrast, when IGF-I was immobilized on a solid phase and reacted with antibody 16G4 which cross-reacts with IGF-II (white circles), IGF-I reacted with antibody 16G4 in a concentration-dependent manner, but the IGF-I [30-41] fragment immobilized on the solid phase (white squares) did not react at all with antibody 16G4. In other words, this demonstrated that the antigen recognition site of the IGF-I-specific antibody 27E10 is in the C-domain of IGF-I, while the antigen recognition site of the IGF-II-cross-reacting antibody 16G4 is at a location other than the C-domain.

As clearly shown in Fig. 3, antibody 27E10 reacts with the C-chain peptide in a concentration-dependent manner, and the epitope of the antibody is the C-chain of IGF-I. Furthermore, the same results were obtained for the monoclonal antibody produced by hybridoma 17B2, showing that the epitope of this antibody is also the C-chain of IGF-I. In addition, in the double-antibody method used in Example 6 below, antibody 17B2 immobilized on a solid phase did not react when biotinated antibody 213D9 was used as the second antibody, as also occurred when biotinated antibody 17B2 was used as the second antibody. Conversely, antibody 213D9 immobilized on a solid phase also did not react when biotinated antibody 17B2 was used as the second antibody, as also occurred when biotinated antibody 213D9 was used as the second antibody. This clearly demonstrated that antibody 17B2, and both antibodies 213D9 and 27E10, recognize the C-chain of IGF-I, and indeed recognize the same epitope on the C-chain. Since antibody 25D4 may be used in the double antibody assay system described in Example 6 below, it clearly recognizes a different epitope than that of antibody 17B2 and antibody 213D9.

### Example 5: Preparation of biotinated antibodies

The biotination of the monoclonal antibodies was conducted according to the method described in New Biochemical Experiments Course, Vol.12 (ed. Japan Biochemistry Association, Tokyo Chemistry Society, 1992) pp. 103 - 104 and Antibodies, A Laboratory Manual (ed. Ed Harlow and David Lane, Cold Spring Harbor Laboratory, 1988) p. 341. That is, 1 mg of antibody 25D4 prepared by the same technique as in Example 3 was dissolved in 1 ml of D'-PBS(-), and 200 µl of 1 M NaHCO₃, was added to pH 9.0. To this solution was then added 25 µl of a biotinating reagent [N-hydroxysuccinimide (Sigma, code B2643) dissolved in dimethylsulfoxide to a concentration of 10 mg/ml) and the mixture was shaken at 4°C for 16 hours. After addition of 20 µl of 1 M NH₄Cl, incubation at room temperature for 10 minutes and suspension of the reaction, the reaction solution was passed through a Sephadex G-25 minicolumn (PD-10) equilibrized with D'-PBS(-), and the protein fraction was collected to prepared the biotinated antibody. Antibody 16G4 was biotinated in the same manner.

### Example 6: Construction of double-antibody assay (sandwich assay) system

### a. Preparation of antibody plate

A double-antibody assay (sandwich assay) system was constructed according to p.580-581 of Antibodies, A Laboratory Manual, mentioned above. First, an antibody plate was prepared by the method described in Evaluation Example 4. That is, antibody 27E10 obtained in Example 3 was dissolved in a coating buffer solution at 10 µg/ml, and dispensed into a 96-well EIA plate (Costar, code 3590) at 100 µl/well and then incubated at 37°C for one hour. After drawing out the solution, the plate was washed 3 times with washing solution. A blocking solution was then added at 150 µl/well and incubation was continued at 37°C for 30 minutes. After removing the blocking solution from each well, the plate was washed 3 times with washing solution to complete preparation of the antibody plate.

### b. Antigen-antibody reaction (1)

rh-IGF-I (Amersham, code ARM4050) was dissolved in a blocking solution to concentrations of 200, 100, 50 and 12.5 ng/ml. These solutions were added to the wells at 100 µl/well and incubated at 37°C for one hour to react the antigen with the immobilized antibody. After the reaction, the supernatant was removed and washing was performed 3 times.

### c. Antigen-antibody reaction (2)

To the IGF-I immobilized by the first antibody immobilized in the wells, there was added a D'-PBS(-) solution containing 10 µg/ml of the biotinated antibody 16G4 prepared in Example 5 at 100 µl/well, and this was incubated at 37°C for one hour to react the second antibody with the antigen immobilized by the first antibody immobilized in the wells. After the reaction, the supernatant was removed and washing was performed 3 times.

### d. Biotin-avidin reaction

An alkaline phosphatase-labelled avidin solution (Dacopatz, code D365, 500-fold dilution) was added at 100 µl/well, and after incubation at 37°C for 30 minutes, the supernatant was removed and the plate was washed 3 times with washing solution.

### e. Alkaline phosphatase reaction

A substrate solution [PNPP dissolved to a concentration of 1 mg/ml in a 10 mM diethanolamine buffer solution (pH 9.5) containing 0.5 mM MgCl₂] was added at 100 µl/well and the plate was allowed to stand at room temperature for 30 minutes, after which a reaction-suspending solution (3N NaOH) was added at 50 µl/well to suspend the enzyme reaction, and an automatic absorbance meter was used to measure the absorbance at 405 nm.

### f. Drawing of standard curve

A double-antibody assay was also performed in the same manner as above using antibody 213D9 as the first antibody and using biotinated antibody 22G4 as the second antibody, and the data obtained from the 4 assays were plotted on a graph with a Y-axis of absorbance and an X-axis of sample concentration, to prepare the standard curve shown in Fig. 4. The black circles represent data obtained using 27E10 as the first antibody and biotinated antibody 16G4 as the second antibody, the white circles represent data obtained using 27E10 as the first antibody and biotinated antibody 22G4 as the second antibody, the black squares represent data obtained using 213D9 as the first antibody and biotinated antibody 16G4 as the second antibody, and the white squares represent data obtained using 213D9 as the first antibody and biotinated antibody 22G4 as the second antibody. These results show that the most suitable combination is 27E10 as the first antibody and biotinated antibody 16G4 as the second antibody, and the linearity of the standard curve shows that the range of measurable concentration is 1-100 ng/ml.

Furthermore, of the antibodies of the present invention, the anti-IGF-I-specific antibodies 17B2, 27E10 and 213D9 are monoclonal antibodies with epitopes in the IGF-1 C-domain, and thus it is possible to construct a double-antibody assay (sandwich assay) system according to the method in Example 5 using any of these as a first antibody in combination with another monoclonal antibody which has an epitope other than the IGF-I C-domain (for example, antibody 25D4 or antibody 16G4) as the biotinated second antibody. Also, if the combination is of two types of antibodies of different classes or subclasses, and they are combined with biotinated anti-mouse Ig antibody specific to the class or subclass of the second antibody, then it is possible to construct a double-antibody assay (sandwich assay) system without biotination of the monoclonal antibody.

### INDUSTRIAL APPLICABILITY

According to the present invention there are provided IGF-I-specific monoclonal antibodies and an assay method for IGF-I by the ELISA method using the antibodies. Since the ELISA method does not employ radioactive isotopes, it has the effect of simplifying the assay of IGF-I. Moreover, the antibodies are highly specific to IGF-I, and therefore the assay may be conducted irrespective of similar growth factors such as IGF-II, insulin and proinsulin. As the concentration of IGF-I in normal human blood has been reported to be about 200 ng/ml (Igaku no Ayumi, Vol. 165, No. 5, p. 268, 1993), the ELISA method employing these antibodies will be quite useful in the clinic.

Furthermore, since the present invention provides different monoclonal antibodies with different epitopes, one may be used as a first antibody and another monoclonal antibody with a different epitope may be biotinated and used as a second antibody, to construct a double-antibody assay (sandwich assay) system. In addition, by combining 2 different antibodies with different epitopes and class or subclass, biotinated anti-mouse Ig antibody specific to the class and subclass of the second antibody may be combined therewith to construct a double-antibody assay (sandwich assay) system without biotination of the monoclonal antibody. The dilution factor of the sample may also be examined to further improve the detection limit.

The monoclonal antibodies obtained according to the present invention may be labelled by a conventional method using any of a variety of different labelling types, such as enzyme labelling, biotin labelling, fluorescent labelling or metal colloid labelling, and thus may be used as important reagents for immunohistochemical research on IGF-I using the fluorescent antibody method, a FACS (Fluorescence Activating Cell Sorter), or the like.
References for microorganisms deposited in conformance with Regulation 13.2, and depositary institution
- Depositary Institute:: National Institute of Bioscience and Human Technology Agency of Industrial Technology and Science
- Address:: 1-3, Higashi 1-chome, Tsukuba-shi, Ibaraki (305) JAPAN

(1) Hybridoma 17B-2
   Deposit No.: FERM BP-4572
   Deposit date: February 17, 1994
(2) Hybridoma 213D-9
   Deposit No.: FERM BP-4570
   Deposit date: February 17, 1994
(3) Hybridoma 27E-10
   Deposit No.: FERM BP-4827
   Deposit date: October, 7, 1994
(4) Hybridoma 25D-4
   Deposit No.: FERM BP-4571
   Deposit date: February, 17, 1994
(5) Hybridoma 16G-4
   Deposit No.: FERM BP-4826
   Deposit date: October, 7, 1994

## Claims

1. A monoclonal antibody which, in immunoreactive methods, reacts specifically with the C-chain of insulin-like growth factor-I but does not substantially react with insulin-like growth factor-II, insulin or proinsulin.

2. A monoclonal antibody according to Claim 1 which is of the antibody class IgG1 and has a κ-chain.

3. A monoclonal antibody according to Claim 1 which is of the antibody class IgG2b and has a κ-chain.

4. A monoclonal antibody according to Claim 1 which is producible by hybridoma 17B-2 (FERM BP-4572), hybridoma 27E-10 (FERM BP-4827), hybridoma 213D-9 (FERM BP-4570) or a cell line derived therefrom.

5. A monoclonal antibody which is producible by hybridoma 25D-4 (FERM BP-4571), hybridoma 16G-4 (FERM BP-4826) or a cell line derived therefrom and which reacts with an epitope other than the C-chain of insulin-like growth factor-I and an epitope in common with IGF-II.

6. A cell line which is hybridoma 17B-2 (FERM BP-4572), hybridoma 27E-10 (FERM BP-4827), hybridoma 213D-9 (FERM BP-4570) or a cell line derived therefrom.

7. A cell line which is hybridoma 25D-4 (FERM BP-4571), hybridoma 16G-4 (FERM BP-4826) or a cell line derived therefrom.

8. An immunochemical assay method for insulin-like growth factor-I which comprises at least the following steps of:
(1) coating insulin-like growth factor-I on a solid-phase surface which is to be used for the immunoreaction;
(2) pre-reacting a test sample with one type of monoclonal antibody selected from monoclonal antibodies according to Claims 1 to 4 as a first antibody;
(3) adding to the system the test sample pre-reacted with the first antibody and allowing the unreacted first antibody to react with the insulin-like growth factor-I immobilized on the solid-phase surface;
(4) reacting the first antibody immobilized via the insulin-like growth factor-I immobilized on the solid-phase surface, with a second antibody which is biotin-labelled antibody specific to the class or subclass of the first antibody;
(5) reacting the biotin portion of the second antibody immobilized via the first antibody and the insulin-like growth factor-I immobilized on the solid-phase surface, with a labelled enzyme which is avidin or streptoavidin labelled with alkaline phosphatase or peroxidase;
(6) adding a substrate to the immobilized labelled enzyme for enzyme reaction to count the amount of labelling; and
(7) measuring the concentration of insulin-like growth factor-I in the test sample by comparing the labelling count of the test sample with a standard curve for previously measured insulin-like growth factor-I.

9. A reagent used for immunochemical assay of insulin-like growth factor-I according to Claim 8, which comprises a monoclonal antibody according to any of Claims 1 to 4.

10. An immunochemical assay method for insulin-like growth factor-I which comprises at least the following steps of:
(1) coating one type of monoclonal antibody selected from monoclonal antibodies according to Claims 1 to 4 as a first antibody on a solid-phase surface;
(2) adding a test sample to the system and reacting the immobilized first antibody with insulin-like growth factor-I in the sample;
(3) reacting the insulin-like growth factor-I immobilized via the first antibody immobilized on the solid-phase surface, with a second antibody which is an antibody according to Claim 5;
(4) reacting the second antibody immobilized via the insulin-like growth factor-I and the first antibody immobilized on the solid-phase surface, with a third antibody which is biotin-labelled anti-mouse IgG subclass antibody specific only to the second antibody;
(5) reacting the biotin portion of the third antibody immobilized via the second antibody, the insulin-like growth factor-I and the first antibody immobilized on the solid-phase surface, with a labelled enzyme which is avidin or streptoavidin labelled with alkaline phosphatase or peroxidase;
(6) adding a substrate to the immobilized labelled enzyme for enzyme reaction to count the amount of labelling; and
(7) measuring the concentration of insulin-like growth factor-I in the test sample by comparing the labelling count of the test sample with a standard curve for previously measured insulin-like growth factor-I.

11. An immunochemical assay method for insulin-like growth factor-I which comprises at least the following steps of:
(1) coating a monoclonal antibody according to Claim 5 as a first antibody on a solid-phase surface;
(2) adding a test sample to the system and reacting the immobilized first antibody with insulin-like growth factor-I in the sample;
(3) reacting the insulin-like growth factor-I immobilized via the first antibody immobilized on the solid-phase surface, with a second antibody which is one type of monoclonal antibody selected from monoclonal antibodies according to Claims 1 to 4;
(4) reacting the second antibody immobilized via the insulin-like growth factor-I and the first antibody immobilized on the solid-phase surface, with a third antibody which is biotin-labelled anti-mouse IgG subclass antibody specific only to the second antibody;
(5) reacting the biotin portion of the third antibody immobilized via the second antibody, the insulin-like growth factor-I and the first antibody immobilized on the solid-phase surface, with a labelled enzyme which is avidin or streptoavidin labelled with alkaline phosphatase or peroxidase;
(6) adding a substrate to the immobilized labelled enzyme for enzyme reaction to count the amount of labelling; and
(7) measuring the concentration of insulin-like growth factor-I in the test sample by comparing the labelling count of the test sample with a standard curve for previously measured insulin-like growth factor-I.

12. An immunochemical assay method for insulin-like growth factor-I which comprises at least the following steps of:
(1) coating one type of monoclonal antibody selected from monoclonal antibodies according to Claims 1 to 4 as a first antibody on a solid-phase surface;
(2) adding a test sample to the system and reacting the immobilized first antibody with insulin-like growth factor-I in the sample;
(3) reacting the insulin-like growth factor-I immobilized via the first antibody immobilized on the solid-phase surface, with a second antibody which is a monoclonal antibody according to Claim 5 which has been biotin-labelled;
(4) reacting the biotin portion of the second antibody immobilized via the insulin-like growth factor-I and the first antibody immobilized on the solid-phase surface, with a labelled enzyme which is avidin or streptoavidin labelled with alkaline phosphatase or peroxidase;
(5) adding a substrate to the immobilized labelled enzyme for enzyme reaction to count the amount of labelling; and
(6) measuring the concentration of insulin-like growth factor-I in the test sample by comparing the labelling count of the test sample with a standard curve for previously measured insulin-like growth factor-I.

13. An immunochemical assay method for insulin-like growth factor-I which comprises at least the following steps of:
(1) coating a monoclonal antibody according to Claim 5 as a first antibody on a solid-phase surface;
(2) adding a test sample to the system and reacting the immobilized first antibody with insulin-like growth factor-I in the sample;
(3) reacting the insulin-like growth factor-I immobilized via the first antibody immobilized on the solid-phase surface, with a second antibody which is one type of monoclonal antibody selected from monoclonal antibodies according to Claims 1 to 4 which has been biotin-labelled;
(4) reacting the biotin portion of the second antibody immobilized via the insulin-like growth factor-I and the first antibody immobilized on the solid-phase surface, with a labelled enzyme which is avidin or streptoavidin labelled with alkaline phosphatase or peroxidase;
(5) adding a substrate to the immobilized labelled enzyme for enzyme reaction to count the amount of labelling; and
(6) measuring the concentration of insulin-like growth factor-I in the test sample by comparing the labelling count of the test sample with a standard curve for previously measured insulin-like growth factor-I.

14. A kit to be used for immunochemical assay of insulin-like growth factor-I according to Claim 10 or Claim 11, which comprises 2 types of monoclonal antibodies, namely one type of monoclonal antibody selected from monoclonal antibodies according to Claims 1 to 4 and one type of monoclonal antibody selected from monoclonal antibodies according to Claim 5.

15. A kit to be used for immunochemical assay of insulin-like growth factor-I according to Claim 12 or Claim 13, which comprises 2 types of monoclonal antibodies, namely one type of monoclonal antibody selected from monoclonal antibodies according to Claims 1 to 4 and one type of monoclonal antibody selected from monoclonal antibodies according to Claim 5, one of which has been biotinated.
